(19) Europäisches Patentamt
European Patent Office
Office européen des brevets

(11) **EP 2 279 776 B1**

(12) **EUROPÄISCHE PATENTSCHRIFT**

(45) Veröffentlichungstag und Bekanntmachung des Hinweises auf die Patenterteilung:
**05.06.2013 Patentblatt 2013/23**

(51) Int Cl.:
***A61N 5/10*** *(2006.01)*   *B01L 3/00* *(2006.01)*

(21) Anmeldenummer: **10007706.4**

(22) Anmeldetag: **24.07.2010**

(54) **Phantom zu experimentellen in-vitro-Validierung von Bestrahlungen unter Bewegungseinfluss unter Berücksichtigung der biologisch effektiven Dosis**

Phantom for experimental in-vitro validation of irradiation under the influence of motion taking the biologically effective dose into consideration

Fantôme destiné à la validation in vitro expérimentale de rayonnements sous l'influence de mouvements en fonction de la dose biologique effective

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **29.07.2009 DE 102009035231**

(43) Veröffentlichungstag der Anmeldung:
**02.02.2011 Patentblatt 2011/05**

(73) Patentinhaber: **GSI Helmholtzzentrum für Schwerionenforschung GmbH 64291 Darmstadt (DE)**

(72) Erfinder:
• **Bert, Christoph 63741 Aschaffenburg (DE)**

• **Gemmel, Alexander 55118 Mainz (DE)**
• **Rietzel, Eike 64331 Weiterstadt (DE)**

(74) Vertreter: **Rück, Dorothee Maria GSI Helmholtzzentrum für Schwerionenforschung GmbH Planckstraße 1 D-64291 Darmstadt (DE)**

(56) Entgegenhaltungen:
**WO-A1-2007/104520   WO-A2-2007/012147 WO-A2-2008/008149   DE-A1-102007 045 879 US-A1- 2008 011 946   US-A1- 2008 298 540**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Phantom zur experimentellen in-vitro-Validierung von Bestrahlungen unter Bewegungseinfluß unter Berücksichtigung der biologisch effektiven Dosis, insbesondere für therapeutische Bestrahlungen an einer Partikeltherapie-Beschleunigeranlage. Die Erfindung betrifft ferner eine Phantomvorrichtung mit einem derartigen Phantom und einer oder mehrerer Bewegungseinrichtungen und ein entsprechendes Verfahren zur Validierung der Bestrahlungen unter Bewegungseinfluss.

Bei der Behandlung von Tumoren im Allgemeinen kommen operative Resektionen, Strahlen- und Chemotherapie zum Einsatz oder eine Kombination aus diesen Verfahren. Bei der Strahlentherapie ist es das Ziel der Behandlung, eine hohe lokale Tumordosis bei möglichst minimaler Belastung des umliegenden Normalgewebes zu erzielen. Hierzu wird die Energie- bzw. Dosisdeposition der Strahlung möglichst konform an den Tumor angepasst. In letzter Zeit werden gute Therapieerfolge mit Bestrahlung durch Ionen statt Photonen erzielt, da die Energie- bzw. Dosisdeposition als Funktion der Eindringtiefe ein scharfes Maximum (sogenannter Bragg-Peak) aufweist. Ein bekanntes Verfahren ist die Strahlapplikation mit passiven Strahlformungskomponenten (u.a. Streufolien, Modulatoren, Kollimatoren, Kompensatoren). Alternativ ist es jedoch möglich, den Ionenstrahl präzise zu fokussieren und den Tumor mit einem nadelfeinen Strahl, einem sogenannten "Pencil-Beam" dreidimensional abzuscannen (Raster-Scan-Verfahren, Spot-Scan-Verfahren, Continous-Scan-Verfahren). Beim Raster-Scan-Verfahren verbleibt der Strahl für eine definierte Teilchenanzahl auf einer Rasterposition und bleibt beim Wechsel zur nächsten Rasterposition angeschaltet. Beim Spot-Scan-Verfahren wird der Strahl zwischen den Rasterpositionen abgeschaltet und beim Continous-Scan-Verfahren wird der Strahl ohne Verweilen auf den Rasterpostionen mit einer optimierten Magnetstromsequenz kontinuierlich über diese gefahren. Neben Protonen werden derzeit Ionen der zweiten Periode des Periodensystems, insbesondere Kohlenstoff-Ionen verwendet. Teilweise werden auch Neon-Ionen verwendet. Der Einsatz dieser Ionen zeichnet sich durch eine erhöhte relative biologische Wirksamkeit (RBW) gegenüber Photonen und auch Protonen bei der Inaktivierung von Zellen aus. Aufgrund ihrer Abhängigkeit vom Dosislevel, dem Gewebetyp und vor allem der Teilchensorte und -energie führt die relative biologische Wirksamkeit der Ionen zu einem zusätzlichen therapeutischen Nutzen im Tumorbereich.

In den letzten Jahren wurde ein großer klinischer Erfolg mit Bestrahlungen im Raster-Scan-Verfahren mit Kohlenstoffionen und einer dedizierten Bestrahlungsplanung erzielt. Vorteile dieses Verfahrens sind der weitgehende Verzicht auf Absorbermaterialien zur Vermeidung der Erzeugung von Sekundärteilchen und vor allem die gute Konformität der erzeugten Dosisverteilungen, insbesondere proximal des Tumors.

Anfangs wurden hauptsächlich Tumore im Schädelbasisbereich und entlang der Wirbelsäule behandelt, deren Bewegung man durch stereotaktische Fixierung auf ein vernachlässigbares Minimum reduzieren kann. Mit der geplanten breiteren klinischen Anwendung des Raster-Scan-Verfahrens in diversen Therapiezentren sollen jedoch auch andere Tumore mit Kohlenstoffionen-Strahlen im Raster-Scan-Verfahren bestrahlt werden. Tumore im Rumpfbereich des Körpers unterliegen jedoch einer stärkeren Bewegung, insbesondere durch die Atmung des Patienten, bei der sich der gesamte Brustkorb bewegt und verformt, ggf. sogar durch den Herzschlag des Patienten. Bei der Behandlung bewegter Tumore oder allgemein bewegter Zielvolumina mit dem Raster-Scan-Verfahren steht man vor der Herausforderung, dass sich diese Bewegung ungünstig auf die Homogenität der Energiedeposition der Kohlenstoffionen im Gewebe auswirken kann. Experimente mit Phantomen haben gezeigt, dass bei der Applikation eines gescannten Strahls Über- und Unterdosierungen im Zielvolumen auftreten können, so dass eine einfache Erweiterung des Zielvolumens um das Ausmaß der Bewegung, wie sie bei passiver Strahlapplikation eingesetzt wird, keine optimale Behandlung zulässt.

Um den Einfluss der Bewegung bei gescannter Strahlapplikation zu korrigieren, werden derzeit entweder die Bestrahlung unter Verwendung von Sicherheitssäumen, Mehrfachbestrahlung, unterbrochene Bestrahlung, bewegungskompensierte Bestrahlung oder Kombinationen der genannten Methoden/Verfahren untersucht und in präklinischen Untersuchungen eingesetzt. Bei der bewegungskompensierten Bestrahlung wird die Strahlposition fortwährend an die Tumorbewegung angepasst. Hierbei wird die Strahllage lateral zur Strahlrichtung und ggf. die Teilchenreichweite kontinuierlich an die Tumorbewegung angepasst. Diesbezüglich wird auf die Dissertationen von S.O. Grötzinger, "Volume Conformal Irradiation of Moving Target Volumes with scanned ionbeams", TU Darmstadt, 2004 und C. Bert "Bestrahlungsplan für bewegte Zielvolumina in der Tumortherapie mit gescanntem Kohlenstoffstrahl", TU Darmstadt, 2006 verwiesen, welche hiermit durch Referenz inkorporiert werden. Jedenfalls ist die bewegungskompensierte Raster-Scan-Ionenstrahlapplikation dem Fachmann auf dem Gebiet der Partikelstrahl-Tumortherapie grundsätzlich bekannt.

Die genannten Dissertationen beschäftigten sich jedoch hauptsächlich mit der physikalischen Energiedeposition der Ionenstrahlen und ließen die erhöhte biologische Wirksamkeit außer Betracht.

Die DE 10 2007 045 879 der Siemens AG und der Anmelderin, beschreibt ein Verfahren und eine Vorrichtung zur Bestrahlung bewegter Zielvolumina. Ziel der vorliegenden Erfindung ist es unter anderem die Sicherheit, Zuverlässigkeit und Qualität solcher Verfahren weiter zu verbessern. Somit besteht weiterer Entwicklungsbedarf zur Verbesserung der Therapieplanung für bewegte Zielvolumina bei der Verwendung gescannter Partikelstrahlen.

Die internationale Patentanmeldung Nr. WO 2007/104520 A1 offenbart einen ortsauflösenden biologischen Detektor als Bestrahlungsverificationsvorrichtung.

Die US Patentanmeldung US 2008/0298540 A1 offenbart ein verfombares Phantom zur Bestrahlungsverification.

Die Offenlegungsschrift DE 10 2007 045 879 A1 offenbart eine Vorrichtung zur Bestrahlung eines sich bewegenden Zielvolumen aufweisend bewegliche Absorberkeile zur Energievariation des Teilchenstrahls.

Es ist daher eine Aufgabe der Erfindung ein Phantom bzw. eine Phantom-Vorrichtung und ein Verfahren bereit zu stellen, mit welchen eine Qualitätssicherung und Validierung der Partikelstrahl-Tumortherapie bei sich während der Bestrahlung bewegenden Tumoren erzielt werden können.

Eine spezifischere Aufgabe der Erfindung ist es, den Algorithmus zur Berechnung der biologisch effektiven Dosis in der Bestrahlungsplanung der Partikelstrahl-Tumortherapie auch für Tumore im Brustkorbbereich mit hoher Qualität und Genauigkeit zu validieren.

Eine weitere spezifische Aufgabe der Erfindung ist es, die Bestrahlungsplanung der Partikelstrahl-Tumortherapie mit bewegungskompensiertem Scan-Verfahren zu verbessern.

Eine weitere spezifische Aufgabe der Erfindung ist es, das Bewegungskompensationssystem der Partikelstrahl-Tumortherapie zu validieren.

Die Aufgabe der Erfindung wird durch den Gegenstand der unabhängigen Patentansprüche gelöst. Weiterbildungen der Erfindung sind Gegenstand der abhängigen Patentansprüche.

Erfindungsgemäß wird eine Phantom-Vorrichtung zur experimentellen Validierung von Bestrahlungen unter Bewegungseinfluss unter Berücksichtigung der biologisch effektiven Dosis, insbesondere für therapeutische Bestrahlungen bereit gestellt. Hiermit kann unter anderem die Berechnung der biologisch effektiven Dosis, d.h. die Berechnung der sogenannten RBW-gewichteten Dosis (RBW = relative biologische Wirksamkeit, engl. RBE = relative biological effectiveness) in der Bestrahlungsplanung für therapeutische Bestrahlungen eines Zielvolumens in einem lebenden Körper mit einem energetischen Strahl validiert werden. Die Validierung erfolgt in-vitro, also in einem Phantom, das einen lebenden biologischen Körper, d.h. einen zu bestrahlenden Patienten (Mensch oder Tier) mit lebenden Testzellen simuliert.

Es geht insbesondere um die Partikeltherapie von Tumoren, d.h. der energetische Strahl ist insbesondere ein Partikelstrahl. Die Partikel sind vorzugsweise Ionen, derzeit insbesondere Kohlenstoffionen, Neonionen oder Protonen, es können jedoch auch andere Partikel wie z.B. andere Ionen, Antiprotonen, Pionen oder andere Hadronen sein. Daher wird manchmal auch von Ionentherapie oder Hadronentherapie gesprochen. Die Phantom-Vorrichtung wird somit am Behandlungsplatz einer Partikeltherapie-Beschleunigeranlage, wie sie z.B. derzeit an der Universitätsklinik in Heidelberg fertig gestellt wird (Heidelberger Ionenstrahl-Therapie), positioniert und dort bestrahlt, um die gesamte Behandlungskette zu validieren. Hierbei können je nach Anforderung der im Vorfeld der Bestrahlung erstellte Bestrahlungsplan, die zeitaufgelöste Computertomographie (4D-CT) oder zeitaufgelöste Magnetresonanztomographie (4D-MRT), der Bestrahlungsvorgang selbst, Verfahren zur Reduzierung des Bewegungseinflusses und/oder die Algorithmen zur Berechnung der RBW-gewichteten Dosis validiert werden.

Die Phantom-Vorrichtung umfasst ein zu bestrahlendes Phantom, welches einen ersten biologischen Dosis-Detektor mit einer ersten biologischen Probe zum Züchten adhärent anwachsender Zellen umfasst. Die erste biologische Probe wiederum weist eine Mehrzahl von Zucht- und Bestrahlungselementen, z.B. Näpfchen einer Mikrotiterplatte, mit jeweils separaten biologischen Zellkulturen auf, so dass auf der ersten biologischen Probe eine Mehrzahl von separaten Zellkulturen gezüchtet und bestrahlt werden können und nachfolgend die Überlebensrate jeweils für die Zellkultur jedes einzelnen Zucht- und Bestrahlungselements unabhängig voneinander bestimmt werden kann. Die biologische Probe kann ebenfalls Gewebeproben, die ein Verbund von Zellkulturen sind, umfassen. Die einzelnen Gewebeproben sind hierbei in einer Matrixstruktur, beispielsweise schachbrettartig, angeordnet. Somit ist der erste biologische Detektor als ortsauflösender biologischer Detektor ausgebildet, wobei jedes Zucht- und Bestrahlungselement sozusagen ein "biologisches Detektorpixel" bildet und die Überlebensrate der Zellkulturen für jedes biologische Detektorpixel separat ausgewertet werden kann. Hierzu werden die Zellkulturen nach der Bestrahlung von der biologischen Probe abgelöst und in einer dem Fachmann grundsätzlich bekannten Weise weiter prozessiert.

Die Phantom-Vorrichtung umfasst ferner eine erste Bewegungseinrichtung für den ersten biologischen Detektor mittels welcher der erste biologische Detektor mit vordefinierter Trajektorie bewegbar ist, um die Bewegung des Zielvolumens mit dem Phantom zu simulieren. Mit anderen Worten ein Bereich des ersten biologischen Detektors repräsentiert das zu bestrahlende Zielvolumen, somit das Volumen in welches der Bragg-Peak gesetzt wird, insbesondere also einen zu bestrahlenden Tumor. Der erste biologische Detektor ist beispielsweise mit einstellbarer, dreidimensionaler Amplitude und/oder Periode und/oder Bewegungsphase zu Beginn der Bestrahlung insbesondere transversal bewegbar. Hierbei entsprechen die Amplitude und/oder die Periode der Bewegung des Phantoms vorzugsweise etwa dem Herzschlag oder der Atmung des zu bestrahlenden Patienten, um die Simulation möglichst realistisch zu gestalten. Die Bewegung kann aber auch entlang einer nichtzyklischen Trajektorie, z.B. durch einen Roboter, durchgeführt werden. Hiermit kann beispielsweise die sogenannte "baseline-drift" ("Grundlinienverschiebung") untersucht werden.

Die Phantom-Vorrichtung umfasst noch einen transversal inhomogenen ersten Absorber vor dem ersten biologischen Detektor, d.h. in Bezug auf den Ionenstrahl strahlaufwärts des ersten biologischen Detektors. Der erste Absorber wird mittels einer zweiten Bewegungseinrichtung mit vordefinierter Trajektorie, insbesondere unabhängig von dem ersten biologischen Detektor bewegt. Insbesondere wird der erste Absorber transversal, ggf. mit einstellbarer Amplitude, Anfangsphase und Periode bewegt. Die Bewegung des transversal inhomogenen ersten Absorber bewirkt eine Veränderung oder Modulation der Partikelenergie, so dass unterschiedliche Eindringtiefen, d.h. die Veränderung der longitudinalen Position des Bragg-Peaks durch unterschiedliche Strahltrajektorien in dem vor dem Zielvolumen liegenden Gewebe mit dem Phantom simuliert werden. Somit können die biologischen Effekte auch bei einer longitudinalen Verschiebung des Bragg-Peaks in unterschiedlichen Tiefen mit dementsprechend unterschiedlichen Teilchenspektren untersucht werden.

Insbesondere können die Bewegungen des ersten biologischen Detektors und des ersten Absorbers unabhängig voneinander eingestellt werden, so dass z.B. unterschiedliche Perioden und/oder ein Phasenversatz gewählt werden kann, um die jeweilige Bewegungs- und Bestrahlungssituation bestmöglich zu simulieren. Der erste Absorber kann patientenspezifisch angefertigt werden, falls dies gewünscht ist. Es ist zu beachten, dass der erste Absorber zu dem Phantom gehört und somit zusätzlich zu dem ggf. verwendeten energiemodulierenden Keilsystem der Partikeltherapie-Beschleunigeranlage zur Kompensation von Reichweiteänderungen oder zum Scannen des Tumors in longitudinaler Richtung vorgesehen ist. Der erste Absorber ist strahlabwärts des Energie-modulierenden Keilsystems der Partikeltherapie-Beschleunigeranlage angeordnet und erlaubt eine Energie-Modulation unabhängig von dem energiemodulierenden Keilsystem der Partikeltherapie-Beschleunigeranlage. In einer besonderen Ausführung kann die Bewegung des ersten Absorbers mit der Bewegung des Energie-modulierenden Systems überlagert werden, so dass eine Hardware-Komponente zur Applikation der Reichweiteänderungen ausreichend ist.

Falls gewünscht, können die Bewegungen des ersten biologischen Detektors und des ersten Absorbers unterschiedliche Richtungen haben. Beispielsweise können diese periodische Schwingungen entlang nicht-paralleler Achsen durchführen. Es hat sich nämlich gezeigt, dass sich die Bewegung von Lungentumoren beim Atmen eher an der Bewegung des Zwerchfells orientiert, d.h. eine kraniale/kaudale Komponente besitzt. Die Bewegung des vorgelagerten Gewebes, also insbesondere die Rippen und das Rippenzwischengewebe besitzt neben einer kranialen/kaudalen Komponente auch eine ventrale/dorsale Bewegungskomponente.

Gemäß einer bevorzugten Ausführungsform der Erfindung umfasst der erste Absorber mehrere Bereiche mit höherem Strahlabsorptionsvermögen zwischen welchen Material mit geringerem Strahlabsorptionsvermögen angeordnet ist. Diese können länglich oder stabartig ausgebildet sein. Der erste Absorber ist demnach strukturiert, insbesondere periodisch strukturiert. Dies kann z.B. durch Stäbe, die in einen Materialblock mit geringerem Absorptionsvermögen eingelassen sind, realisiert werden. Die Stäbe mit dem höheren Absorptionsvermögen sind dabei an menschliche oder tierische Knochen und das dazwischen liegende Material an das geringere Absorptionsvermögen von menschlichem oder tierischem Fleisch zwischen den Rippen angepasst, so dass der Bereich in der Patientenbrust zwischen der Oberfläche des Brustkorbs und der Lunge simuliert werden kann, also der Bereich in dem sich unter anderem die Rippen befinden. Mit einem solchen Absorber kann z.B. die Bestrahlung von postcostalen Tumoren, z.B. Lungentumoren, simuliert werden, bei welcher die longitudinale Position des Bragg-Peaks schwanken kann, je nachdem, ob der Strahl z.B. im eingeatmeten Zustand eine Rippe durchquert und im ausgeatmeten Zustand zwischen den Rippen passiert.

Weiter bevorzugt umfasst die Phantom-Vorrichtung einen zweiten biologischen Detektor mit einer zweiten biologischen Probe, wobei der zweite biologische Detektor mittels einer dritten Bewegungseinrichtung bewegbar ist. Insbesondere kann eine Bewegung mit vordefinierter Trajektorie erfolgen, so dass z.B. der zweite biologische Detektor mit vordefinierter Amplitude und Periode transversal bewegt wird.

Bevorzugt ist es, wenn die Bewegungen des ersten und zweiten biologischen Detektors unabhängig voneinander steuerbar (beziehungsweise einstellbar) sind und weiter bevorzugt die biologischen Detektoren relativ zueinander bewegbar sind. Somit können der erste und zweite biologische Detektor relativ zueinander bewegt werden, so dass in vorteilhafter Weise unterschiedliche Bewegungen innerhalb des zu bestrahlenden Körpers simuliert werden können. So kann sich z.B. ein Lungentumor, dessen Gewebe von dem zweiten biologischen Detektor simuliert wird, beim Atmen anders bewegen, als im Strahlengang vor dem Tumor liegendes Normalgewebe. Mit dieser Ausführungsform können also gleichzeitig in dem ersten biologischen Detektor z.B. Zellen, die Normalgewebe vor dem Tumor repräsentieren, d.h. im Plateaubereich vor dem Bragg-Peak der relativen Dosisverteilung und in dem zweiten biologischen Detektor Zellen, die den Tumor repräsentieren, d.h. im Bragg-Peak, bestrahlt werden und nachfolgend die Überlebensraten separat voneinander also tiefenaufgelöst bestimmt werden. Eine Anwendung ist beispielsweise folgende Abfolge bezogen auf den Ionenstrahl: i) risikoträchtiges Organ (sogenanntes "organ at risk" = OAR) im Eingangskanal, ii) der Tumor und iii) ein weiteres OAR distal des Tumors, z.B. Herz oder Mediastinum, um eine solche Organ-/Gewebereihenfolge zu simulieren.

Vorzugsweise umfasst der erste und/oder zweite biologische Detektor jeweils einen Behälter zum Einfüllen von Flüssigkeit, insbesondere Nährmedium oder Nährlösung für die lebenden Zellen oder das Gewebe. Der erste und zweite biologische Detektor umfassen demnach separate Behälter. Die erste und zweite biologische Probe wird in den ersten bzw. zweiten Behälter in das jeweilige Nährmedium eingesetzt. Dies ist besonders vorteilhaft, wenn z.B. unterschiedliche Zellsysteme mit unterschiedlicher Strahlensensitivität in dem ersten und zweiten biologischen Detektor vorhanden sind. Die Nährmedien können an die jeweiligen lebenden Zellen und/oder andere Bestrahlungselemente angepasst sein, können also auch unterschiedlich sein. Ggf. kann noch ein dritter biologischer Detektor mit einer dritten biologischen Probe in einem dritten Behälter mit Nährmedium vorgesehen sein.

Hiermit können unterschiedliche Zelltypen mit unterschiedlichen Nährmedien, z.B. Haut im Eingangskanal, Normalgewebe im Dosisplateau und Tumorgewebe in Zielvolumen (Bragg-Peak) simuliert werden. Ferner können unterschiedliche Oxigenierungslevel in den Behältern eingestellt werden, z.B. um vordefinierte hypoxische Bedingungen im Tumorgewebe, nicht jedoch in dem übrigen Gewebe zu simulieren. Ferner können unterschiedliche Zellzyklen in den verschiedenen biologischen Detektoren eingesetzt werden. Allgemein können also der erste und zweite biologische Detektor unterschiedliche Zellsysteme enthalten.

Bevorzugt ist es, wenn die Vorrichtung ferner einen zweiten transversal inhomogenen Absorber und eine vierte Bewegungseinrichtung umfasst, wobei der erste Absorber, der erste biologische Detektor, der zweite Absorber und der zweite biologische Detektor hintereinander angeordnet sind. Der zweite Absorber ist bevorzugt mittels der vierten Bewegungseinrichtung bewegbar. Eine Bewegung kann insbesondere mit vordefinierter Amplitude und Periode transversal erfolgen, wobei die periodischen Bewegungen der beiden biologischen Detektoren und der beiden Absorber bevorzugt unabhängig voneinander steuerbar sind.

Weiter vorzugsweise ist eine Dreheinrichtung vorgesehen, mittels welcher das Phantom mit vordefinierter Amplitude, Anfangsphase und Periode um eine Achse quer zur Richtung des energetischen Strahls drehbar ist. Hierdurch ist eine Bewegung des Phantoms ermöglicht, welche eine periodische Veränderung des Einstrahlwinkels in das Zielvolumen umfasst. Dies kann vorteilhaft sein, wenn rotatorische Bewegungskomponenten, die z.B. von der Atmung verursacht werden können, simuliert werden sollen. Andererseits kann eine einfache Drehung des Phantoms auch dazu verwendet werden, um Mehrfeld-Bestrahlungen aus unterschiedlichen Richtungen, z.B. für isozentrische Bestrahlungen des Phantoms durchzuführen.

Die Bewegung der biologischen Detektoren und/oder der Absorber kann von eindimensional parallel bis zu sechsdimensional (drei lineare Freiheitsgrade und drei rotatorische Freiheitsgrade) roboterbasiert und Detektor/Absorber-spezifisch durchgeführt werden. Es sind sogar deformierte Absorber, z.B. verformbare Gummiblöcke denkbar. Durch die Ortsauflösung können Dosisabweichungen aufgrund der Wechselwirkung zwischen dem Scannen des Ionenstrahls und des Zielvolumens detektiert werden.

Gemäß einer bevorzugten Ausführungsform ist die erste, zweite und/oder dritte biologische Probe jeweils als eine Zellkultureinrichtung jeweils in Form einer Mikrotiterplatte jeweils mit einer Mehrzahl von Vertiefungen ausgebildet. Die Vertiefungen oder Näpfchen bilden jeweils die Zucht- und Bestrahlungselemente für die biologischen Zellkulturen, bzw. die "biologischen Detektorpixel". Hierzu weisen die Vertiefungen oder Näpfchen eine Oberfläche im Inneren der Näpfchen auf, welche zum Anwachsen der Zellkulturen hergerichtet ist, z.B. eine Zellwachstumsschicht am Boden der Näpfchen, so dass die Zellkulturen adhärent innerhalb der Näpfchen anwachsen können.

Insbesondere ist es möglich, die Phantom-Vorrichtung derart auszubilden, dass die erste biologische Probe als eine erste Mikrotiterplatte mit einer Mehrzahl von Näpfchen ausgebildet ist, wobei die Näpfchen jeweils die Zucht- und Bestrahlungselemente für die biologischen Zellkulturen bilden.

Die Mikrotiterplatten sind vorzugsweise so in den jeweils zugehörigen Behälter für Nährmedium einsetzbar, dass sich die Mikrotiterplatten bei der Bestrahlung quer zur Richtung des energetischen Strahls erstrecken. Wenn die Mikrotiterplatten in den zugehörigen Behälter eingesetzt sind und der jeweilige Behälter mit Nährmedium gefüllt ist, sind die Mikrotiterplatten jeweils beidseits von dem zugehörigen Nährmedium umgeben.

Insbesondere ist es möglich, die Phantom-Vorrichtung derart auszubilden, dass der erste biologische Detektor einen ersten Behälter für Flüssigkeit aufweist, in welchen die erste Mikrotiterplatte einsetzbar ist, derart, dass die erste Mikrotiterplatte beidseits von der Flüssigkeit umgeben ist, wenn die erste Mikrotiterplatte in dem ersten Behälter eingesetzt und der erste Behälter mit der Flüssigkeit gefüllt ist.

Versuche haben gezeigt, dass es vorteilhaft ist, die Mikrotiterplatten nicht zu verschließen, sondern in unverschlossenem Zustand in die mit Nährmedium befüllbaren Behälter einzusetzen. Hierdurch wird die Bildung von Luftblasen in den Näpfchen vermieden, welche die Eindringtiefe bzw. die Position des Bragg-Peaks unkontrolliert beeinflussen können, insbesondere wenn der jeweilige biologische Detektor bewegt wird, da dies sogar ein Schwappen des Nährmediums verursachen könnte. Dementsprechend ist es bevorzugt, wenn die Phantom-Vorrichtung derart ausgebildet wird, dass die erste Mikrotiterplatte unverschlossen ist, wenn sie in dem Behälter mit der Flüssigkeit eingesetzt wird.

Ferner können mehr als eine biologische Probe, z.B. zwei oder mehr Mikrotiterplatten in den ersten und/oder die weiteren Behälter eingesetzt werden, wobei die Mikrotiterplatten parallel hintereinander in dem jeweiligen Behälter angeordnet sind, so dass im selben Behälter zwei oder mehr getrennte Zellkulturen hintereinander bestrahlt werden können. Mit anderen Worten kann es vorteilhaft sein, wenn die Phantom-Vorrichtung derart ausgebildet ist, dass eine zweite biologische Probe in Form einer zweiten Mikrotiterplatte in den ersten Behälter eingesetzt ist, wobei die erste und zweite Mikrotiterplatte parallel hintereinander in dem zweiten Behälter angeordnet sind, so dass der biologische Detektor eine Ortsauflösung in longitudinaler Richtung aufweist. Somit weist dann der jeweilige biologische Detektor auch eine Ortsauflösung in longitudinaler Richtung auf. Erfindungsgemäß können somit beispielsweise Validierungsmessungen für unterschiedliche Schichten des Tumors im Scan-Verfahren, insbesondere Raster-Scan-Verfahren durchgeführt werden. Alternativ können die Mikrotiterplatten eines Behälters mit einem leichten Versatz quer zum Strahl positioniert werden, um eine höhere Ortauflösung zu erreichen.

Gegebenenfalls können unterschiedliche Bereiche des biologischen Detektors unmittelbar nacheinander bestrahlt werden, so dass mit einmaliger Bestükkung des biologischen Detektors mehrere Messungen möglich sind.

Die Phantom-Vorrichtung umfasst.bevorzugt einen Bewegungssensor, welcher die Bewegung des Phantoms erfasst, und eine Steuereinrichtung für die Partikeltherapie-Beschleunigeranlage zur bewegungskompensierten Bestrahlung des Phantoms.

Die erfindungsgemäße Phantomvorrichtung eignet sich grundsätzlich auch für Bestrahlungs-Anlagen mit passiver Strahlanpassung. Besondere Vorzüge zeigt die Erfindung aber in Kombination mit dem bewegungskompensierten Raster-Scan-Verfahren, also für die Validierung der Berechnung der RBW-gewichteten Dosis bei diesem Verfahren. Das bewegungskompensierte Raster-Scan-Verfahren ist dem Fachmann grundsätzlich bekannt. Erfindungsgemäß wird nun das Phantom im Raster-Scan-Verfahren bestrahlt und es werden die Bewegungsdaten des Phantoms zur Bewegungskompensation verwendet. Die Bewegungsdaten werden vorzugsweise mit zumindest einem Bewegungssensor ermittelt und an das Therapiekontrollsystem bzw. die Steuereinrichtung für das Raster-Scannen der Partikeltherapie-Beschleunigeranlage zur bewegungskompensierten Bestrahlung des Phantoms übermittelt.

Für die experimentelle in-vitro-Validierung des Algorithmus zur Berechnung der RBW-gewichteten Dosis wird demnach das Phantom an einem Patientenbestrahlungsplatz, z.B. auf der Patientenliege temporär positioniert und dort bestrahlt. Der erste und die ggf. weiteren biologischen Detektoren sowie ggf. die Absorber des Phantoms werden während der Bestrahlung mit vordefinierter Amplitude und Periode transversal bewegt, um die Bewegung des zu bestrahlenden Zielvolumens oder Tumors mit dem Phantom zu simulieren. Nach der Bestrahlung werden die Überlebensraten der Zellkulturen aus den Zucht- und Bestrahlungselementen jeweils separat bestimmt, so dass eine ortsauflösende Messung mit den "biologischen Detektorpixeln" durchgeführt wird und anhand der Überlebensraten der Zellkulturen der einzelnen Detektorpixel wird der Algorithmus zur Berechnung der RBW-gewichteten Dosis der Partikelbeschleuniger-Therapieanlage überprüft.

Wenn wie bevorzugt ist, der Partikelstrahl im Raster-Scan-Verfahren über das Phantom gescannt wird und der erste biologische Detektor während des Scannens transversal bewegt wird, entsteht eine Überlagerung der Scanbewegung des Strahls und der Bewegung des ersten und der ggf. weiteren biologischen Detektoren in transversaler Richtung. Das Gleiche gilt für die weiteren biologischen Detektoren und ggf. für die Absorber. Für die Validierung werden die Strahlposition und Strahlteilchenanzahl des Raster-Scan-Strahls sowie die Bewegung des ersten biologischen Detektors und ggf. der weiteren biologischen Detektoren, also Signale der Beschleunigeranlage und Signale der Phantom-Vorrichtung, jeweils zeitaufgelöst aufgezeichnet und korreliert ausgewertet. Beispielsweise kann die Phantom-Vorrichtung einen Eingang für ein Signal der Beschleuniger-Anlage, das synchron zur Strahlpulsung (z.B. low/high) ist, welches von der Phantom-Vorrichtung aufgezeichnet wird. Es kann aber auch mit einem Signal der Beschleuniger-Anlage die Bewegung der Phantom-Vorrichtung synchron zum Strahl gestartet werden oder die Phantom-Vorrichtung gibt ein Signal aus, mit welchem die Bestrahlung synchron zur Bewegung gestartet wird. Mittels der korrelierten Auswertung der Strahlposition und Strahlteilchenanzahl zu den Bewegungsdaten des ersten biologischen Detektors können die applizierten Strahlendosen separat für die jeweiligen Zucht- und Bestrahlungselemente (biologische Detektorpixel) ermittelt werden. Insbesondere ist es möglich, die RBW-gewichtete Dosis separat für die jeweiligen Zucht- und Bestrahlungselemente zu ermitteln (RBW steht dabei für "relative biologische Wirksamkeit"). Somit kann experimentell für jedes der Zucht- und Bestrahlungselemente separat die relative biologische Wirksamkeit (RBW) der Bestrahlung bzw. die sogenannte RBW-gewichtete Dosis unter dem Einfluss der Bewegung bestimmt werden. Es kann demnach der Algorithmus zur Berechnung der RBW-gewichteten Dosis anhand der ortaufgelösten Daten der gemessenen RBW-gewichteten Dosis unter dem Einfluss der Bewegung validiert werden. Das Validierungsverfahren kann mit den o.g. Verfahren (Sicherheitssäume, Unterbrochene Bestrahlung, Mehrfachbestrahlung, Bewegungskompensation, Kombinationen der Verfahren) insbesondere mit der Bewegungskompensation der Rasterscan-Bestrahlung kombiniert werden, so dass der Algorithmus zur Berechnung der RBW-gewichteten Dosis auch mit Bewegungskompensation validiert werden kann.

Insbesondere kann die Validierung des Algorithmus zur Berechnung der RBW-gewichteten Dosis in Kombination mit dem dreidimensionalen Scannen durch den Partikelstrahl erfolgen. Beim dreidimensionalen Scannen wird das Zielvolumen typischerweise schichtweise abgerastert, wobei jede Schicht (sogenannte Isoenergie-Schicht) zweidimensional abgerastert wird. Die Tiefenvariation erfolgt in typischen Partikeltherapie-Beschleunigeranlagen entweder durch den Beschleuniger (beispielsweise ein Synchrotron und/oder ein Linearbeschleuniger) oder durch ein passives Absorbersystem mittels welchem die Partikelenergie und damit die Eindringtiefe variiert wird (in Anlagen mit einem Zyklotron). Zusätzlich zu dieser Energiemodulation durch die Partikeltherapie-Beschleunigeranlage umfasst das Phantom den transversal inhomogenen ersten Absorber mittels dessen transversaler Bewegung die zusätzliche und, im Gegensatz zu der Energiemodulation durch das Keil-Modulatorsystem, generell unerwünschte zeitlich veränderliche Beeinflussung der Partikelenergie durch die Bewegung des Körpers, z.B. beim Atmen simuliert wird. Es wird demnach für die Validierung eine Überlagerung der Tiefenmodulation des Rasterscannens durch die Partikeltherapie-Beschleunigeranlage und einer Tiefenmodulation durch den zum Phantom gehörenden ersten Absorber erzeugt. Bei passiver Strahlanpassung sind noch die dem Fachmann grundsätzlich bekannten patientenfeldspezifischen Absorber und Kollimatoren vorgesehen.

Somit wird vorzugsweise das Zielvolumen mit dem Partikelstrahl "pencil beam" abgerastert und die dreidimensionale Rasterung mit einer transversalen, ggf. zweidimensionalen Bewegung des ersten biologischen Detektors und ggf. einer zusätzlichen Variation der Eindringtiefe mittels des zum Phantom gehörenden ersten Absorbers überlagert. Durch die Bewegung des ersten biologischen Detektors und des ersten Absorbers wird demnach eine dreidimensionale Bewegung des Istzielpunktes maximaler Energiedeposition im Phantom im dreidimensionalen Raum zum Sollzielpunkt maximaler Energiedeposition bewirkt. Somit kann der Algorithmus zur Berechnung der RBW-gewichteten Dosis unter dem Einfluss einer dreidimensionalen Relativbewegung zwischen dem Istzielpunkt maximaler Energiedeposition und dem Phantom und der dreidimensionalen Rasterung des Zielvolumens validiert werden.

Bevorzugt kann es insbesondere sein, wenn bei dem Verfahren der Strahl zweidimensional transversal über das Phantom gescannt wird und der erste biologische Detektor während des Scannens zweidimensional transversal bewegt wird, so dass eine Überlagerung der Scan-Bewegung des Strahls und der Bewegung des ersten biologischen Detektors in beiden transversalen Richtungen entsteht.

Weiterhin kann es sich als vorteilhaft erweisen, wenn bei dem Verfahren das Scannen des Strahls über das Phantom eine Energiemodulation des Strahls durch die Beschleunigeranlage umfasst und zusätzlich zu dieser Energiemodulation eine weitere hiervon unabhängige Veränderung der Eindringtiefe des Strahls mittels Energievariation durchgeführt wird, in dem ein zum Phantom gehörender transversal inhomogener Absorber strahlaufwärts der ersten biologischen Probe transversal bewegt wird.

Das vorgeschlagene Phantom zur experimentellen in-vitro-Validierung von Bestrahlungen unter Bewegungseinfluss unter Berücksichtigung der biologisch effektiven Dosis, insbesondere für therapeutische Bestrahlungen an einer Partikeltherapie-Beschleunigeranlage, umfasst vorzugsweise einen ersten biologischen Detektor mit einer ersten biologischen Probe, welche eine Mehrzahl von Zucht- und Bestrahlungselementen mit jeweils separaten biologischen Proben, insbesondere biologischen Zellkulturen aufweist, wobei die erste biologische Probe als eine Platte mit einer Mehrzahl von näpfchenförmigen Vertiefungen ausgebildet ist, wobei die Vertiefungen eine Oberfläche aufweisen, welche zum Anwachsen der biologischen Proben, insbesondere der biologischen Zellkulturen hergerichtet ist, so dass die biologischen Proben, insbesondere biologische Zellkulturen adhärent innerhalb der Vertiefungen anwachsen können.

Insbesondere ist es möglich, das bei dem Phantom die erste Mikrotiterplatte unverschlossen ist, wenn sie in den Behälter mit der Flüssigkeit eingesetzt wird.

Weiterhin ist es bevorzugt, wenn bei dem Phantom der erste biologische Detektor eine zweite Mikrotiterplatte umfasst, welche in den ersten Behälter einsetzbar und wieder entnehmbar ist, wobei die erste und zweite Mikrotiterplatte parallel hintereinander angeordnet sind.

Bevorzugt ist es, wenn das Phantom ferner einen zweiten biologischen Detektor mit einem zweiten Behälter für Flüssigkeit aufweist, in welchen eine weitere Mikrotiterplatte einsetzbar und wieder entnehmbar ist, wobei der erste und zweite Behälter hintereinander angeordnet sind.

Möglich ist es dabei, dass der erste und zweite biologische Detektor unterschiedliche Zellsysteme enthalten.

Weiterhin kann es sich in diesem Zusammenhang als vorteilhaft erweisen, wenn das Phantom weiterhin einen transversal inhomogenen zweiten Absorber umfasst, wobei der erste Absorber, der erste biologische Detektor, ein zweiter Absorber und der zweite biologische Detektor hintereinander angeordnet sind.

Im Folgenden wird die Erfindung anhand von Ausführungsbeispielen und unter Bezugnahme auf die Figuren näher erläutert, wobei gleiche und ähnliche Elemente teilweise mit gleichen Bezugszeichen versehen sind und die Merkmale der verschiedenen Ausführungsbeispiele miteinander kombiniert werden können.

Es zeigen:

Fig. 1    ein Diagramm der relativen Dosis als Funktion der Eindringtiefe für verschiedene Strahlsorten und Strahlenergien,

Fig. 2   ein Diagramm der Dosis als Funktion der Eindringtiefe im Unterschied der physikalischen Dosis und der biologisch effektiven Dosis,

Fig. 3   eine schematische dreidimensionale Darstellung einer Bestrahlungsvorrichtung mit einem Raster-Scan-System für eine Therapiebestrahlungsanlage,

Fig. 4   ein Foto eines Patienten auf einer Patientenliege in Bestrahlungsposition,

Fig. 5   eine schematische Darstellung des Systems zur Bewegungskompensation in einer Bestrahlungsvorrichtung,

Fig. 6   eine schematische Darstellung einer Phantom- Vorrichtung in Kombination mit einem bewegungskompensiertem Partikelstrahl,

Fig. 7   experimentelle Ergebnisse mit der Phantom-Vorrichtung aus Fig. 6,

Fig. 8   eine schematische Darstellung einer Phantom- Vorrichtung bei Verwendung ohne Bewegungskompensation,

Fig. 9   experimentelle Ergebnisse mit dem Aufbau aus Fig. 8,

Fig. 10   eine weitere Ausführungsform einer Phantom- Vorrichtung mit mehreren biologischen Detektoren und mehreren Absorbern,

Fig. 11   eine schematische Darstellung des ersten Absorbers aus Fig. 10,

Fig. 12   einen horizontalen Schnitt durch einen Behälter für die biologischen Detektoren,

Fig. 13   eine Vorderansicht einer Mikrotiterplatte zum Einsetzen in den Behälter aus Fig. 12,

Fig. 14   ein Ablaufschema zur Bestimmung des Zellüberlebens,

Fig. 15   eine schematische Querschnittsdarstellung einer Bewegungseinrichtung.

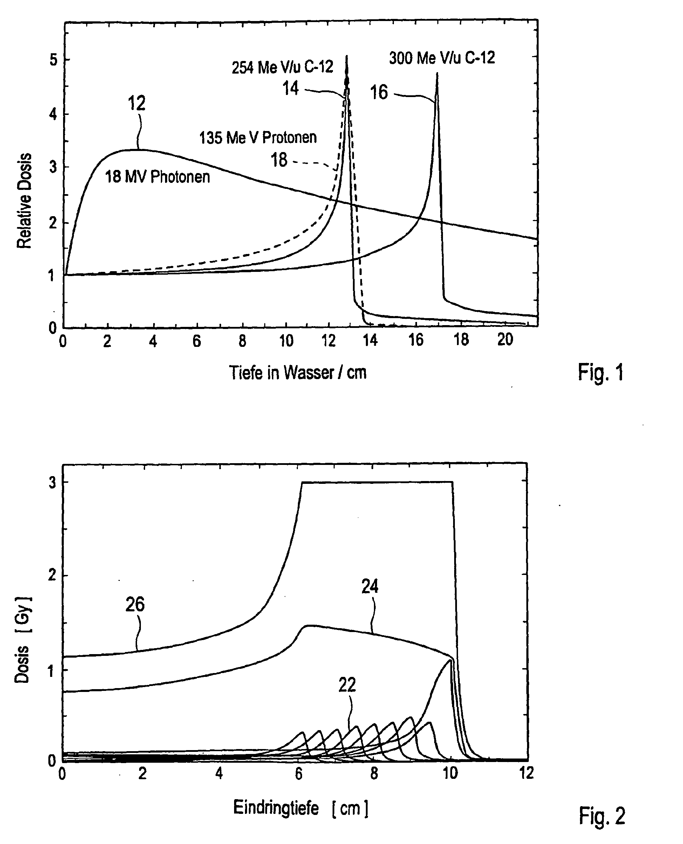

Bezug nehmend auf Fig. 1 ist die Tiefendosisverteilung für verschiedene Strahlarten dargestellt. Im Unterschied zu Photonen (Kurve 12), die nach einem Aufbaueffekt einen exponentiellen Abfall der Dosis mit der Tiefe aufweisen, zeigen Ionen am Ende der Strahlreichweite ein ausgeprägtes Dosismaximum, welches als Bragg-Peak oder Bragg-Maximum bezeichnet wird. Dieses Maximum kann durch Energievariation in der Tiefe verschoben werden. Es ist erkennbar, dass das Bragg-Maximum für Kohlenstoffionen (Kurven 14 und 16) schärfer ist als für Protonen (Kurve 18).

Das Ziel einer therapeutischen Bestrahlung ist die Inaktivierung von Tumorzellen, um das weitere Wachstum und die Vermehrung dieser Zellen zu unterbinden. Hierzu geht man davon aus, dass die Zell-DNS (Desoxyribonukleinsäure) möglichst effektiv geschädigt werden muss, damit die Zelle nicht überlebt. Die Überlebenswahrscheinlichkeit S einer Zelllinie nach Bestrahlung mit einer Dosis D wird üblicherweise durch einen exponentiell linear quadratischen Ansatz beschrieben:

$$S = \exp(-\alpha \cdot D - \beta \cdot D^2)$$

$\alpha$ und $\beta$ sind ein für das Gewebe und die Strahlqualität charakteristisches Parameterpaar, wobei der Quotient $\alpha/\beta$ ein Maß für das Reparaturvermögen der Zelle ist. Bei der Therapie mit Ionen nutzt man nun die starke Abhängigkeit des Reparaturvermögens von der Qualität der Strahlung aus. Diesbezüglich wird von der relativen biologischen Wirksamkeit (RBW) gesprochen. Die RBW ist definiert als der Quotient der Photonendosis und der absorbierten Dosis der Ionenstrahlen, die jeweils notwendig sind, um den gleichen biologischen Effekt zu erzielen. Allgemein ist die Kenntnis der physikalischen Energie-Dosis D nicht gleichzusetzen mit der Kenntnis der RBW-gewichteten Dosis, welche allerdings für eine exakte Bestrahlungsplanung unerlässlich ist, da z.B. eine Überdosierung des Strahls im schlimmsten Fall bis zu letalen Folgen haben könnte. Eine Möglichkeit der theoretischen Berechnung der RBW ist das Local-Effect-Modell, bei welchem die Größe des Zellkerns, die Bahnstruktur des Ions und die Zellüberlebenskurve nach Photonenbestrahlung berücksichtigt wird. Es ist jedoch ersichtlich, dass derartige theoretische Modelle eine Vielzahl von Näherungen enthalten und experimentell überprüft werden sollten. Hier setzt die Erfindung an.

Bezug nehmend auf Fig. 2 ist die Erzeugung eines ausgedehnten Bragg-Peaks dargestellt. Aus der Überlagerung vieler Einzelstrahlen 22 unterschiedlicher Reichweite ergibt sich eine Verteilung der absorbierten physikalischen Dosis 24. Die Kurve 26 stellt eine RBW-gewichtete Dosisverteilung 26 dar.

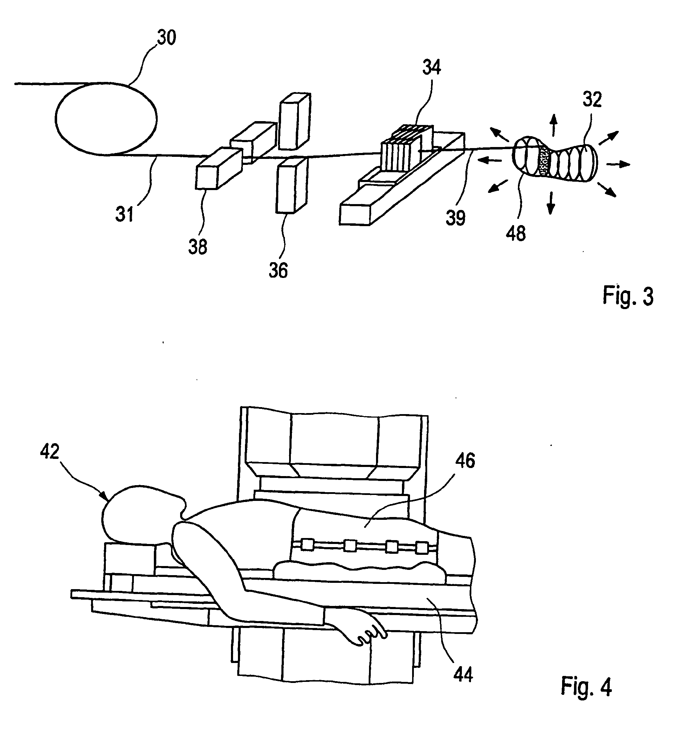

Bezug nehmend auf Fig. 3 wird das Raster-Scan-Verfahren erläutert. Beim Raster-Scan-Verfahren wird der von der Beschleunigeranlage 30 bereit gestellte Ionenstrahl 31 in über ein zu bestrahlendes Zielvolumen 48 gescannt. Das Zielvolumen 48 ist hierbei in Isoenergieschichten 32, d.h. in Schichten gleicher Teilchenreichweite, aufgeteilt. Hierzu steht z.B. am Synchrotron der Anmelderin eine Energiebibliothek zur Verfügung, die 252 verschiedene Energien im Bereich von etwa 88 bis 430 MeV/u umfasst, was einer wasseräquivalenten Reichweite von etwa 2 bis 32 cm bei einer Schrittweite von

1 bis 1,5 mm entspricht. Innerhalb jeder Schicht wird der Strahl mit dem Raster-Scan-Verfahren zeilenweise über ein regelmäßiges Gitter von sogenannten Rasterpunkten gefahren. Die Bestrahlung erfolgt intensitätskontrolliert, d.h. nachdem an einem Rasterpunkt die zuvor festgelegte Anzahl von Teilchen deponiert wurde, wird der Teilchenstrahl ohne Strahlunterbrechung auf die Position des nächsten Rasterpunktes eingestellt. Dabei dauert im Mittel die Bestrahlung eines Rasterpunktes etwa 10 ms, einer Schicht etwa 2 s und einer Fraktion etwa 5 min. Das zweidimensionale Abscannen der Isoenergie-Schichten wird mit zwei zueinander senkrechten Scanner-Magnetpaaren 36, 38 durchgeführt, wie dem Fachmann grundsätzlich bekannt ist. Mit dem Doppelkeil-Modulatorsystem 34 kann die Tiefenanpassung in Zusammenhang mit der Bewegungskompensation erzielt werden.

Ziel der Bestrahlungsplanung ist ganz allgemein einen Machinenparametersatz zu optimieren, der zu einer optimalen Dosisverteilung führt. Dazu gehört die Festlegung der Anzahl der Felder, ihre Einstrahlrichtung, die Definition der Iso-Energieschichten und der Strahlpositionen. Ferner umfasst diese die Optimierung der Teilchenanzahl pro Strahlposition aus der die zu erwartende RBW-gewichtete Dosisverteilung berechnet werden kann, die so optimiert ist, dass eine Zerstörung des Tumors bei gleichzeitiger Schonung des umliegenden, gesunden Gewebes erfolgt, berechnet werden kann. Bei der Ionentherapie mit gescannten Kohlenstoffstrahlen ist ein Feld beispielsweise durch die Position der Patientenliege definiert, ferner durch den Zielpunkt im Patienten und den Bestrahlungsparametern des sogenannten Bestrahlungsplans, der die zu verwendeten Energien, Halbwertsbreiten und Strahlpositionen (als Rasterpunkte) enthält. Jeder Rasterpunkt ist durch seine Isoenergieschicht, zwei Koordinaten, die den horizontalen und senkrechten Auslenkungen des Strahls durch die Scannermagnete 36, 38 entsprechen, und die zu deponierende Teilchenzahl definiert. Eine robotorgesteuerte Patientenlagerung und/oder eine Gantry sind ebenfalls möglich und erlauben jeweils noch mehr Freiheitsgrade.

Fig. 4 zeigt einen Patienten 42 auf einer Behandlungsliege 44, positioniert zur Bestrahlung im Abdomenbereich 46. Es ist ersichtlich, dass wenn der Patient atmet, eine Relativbewegung zwischen dem Tumor und dem Sollrasterpunkt eintritt, da die externe Fixierung des Abdomenbereich 46 nicht zu einer Fixierung der internen, atmungsbedingten Bewegung führt. Derartige Veränderungen der Tumorposition während einer Fraktion, d.h. Bewegungen des Patienten im Sekunden- oder Minutenbereich werden als intrafraktionelle Bewegungen bezeichnet. Insbesondere diese intrafraktionellen Bewegungen überlagern sich mit der Scan-Bewegung des Strahls, was zu grundsätzlich unerwünschter verschlechterter Konformität der räumlichen Dosisverteilung, insbesondere zu lokalen Schwankungen führen kann. Erste Experimente haben ergeben, dass im Rumpfbereich des Menschen die Bewegung in craniokaudaler Richtung bis zu 4 cm am größten ist. Ferner hat sich gezeigt, dass Lungentumoren im unteren Teil der Lunge sich deutlich mehr bewegen als Tumoren im oberen Teil der Lunge. Tumoren, die an festen Strukturen wie den Wirbeln oder der Brustwand anliegen, bewegen sich weniger. Bei vielen Patienten weist die Bewegungstrajektorie des Tumors sogar eine Bahn mit Hysterese auf.

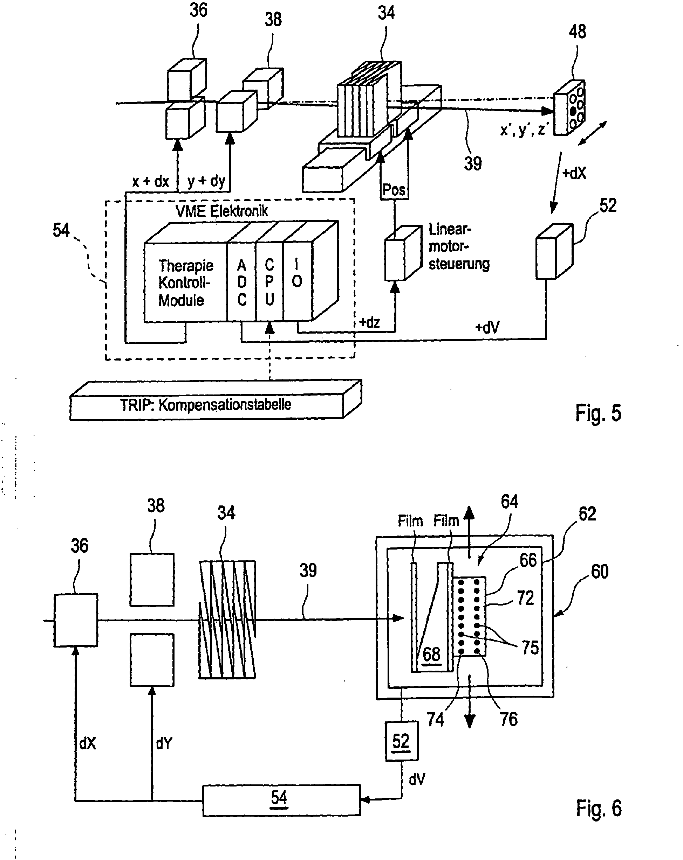

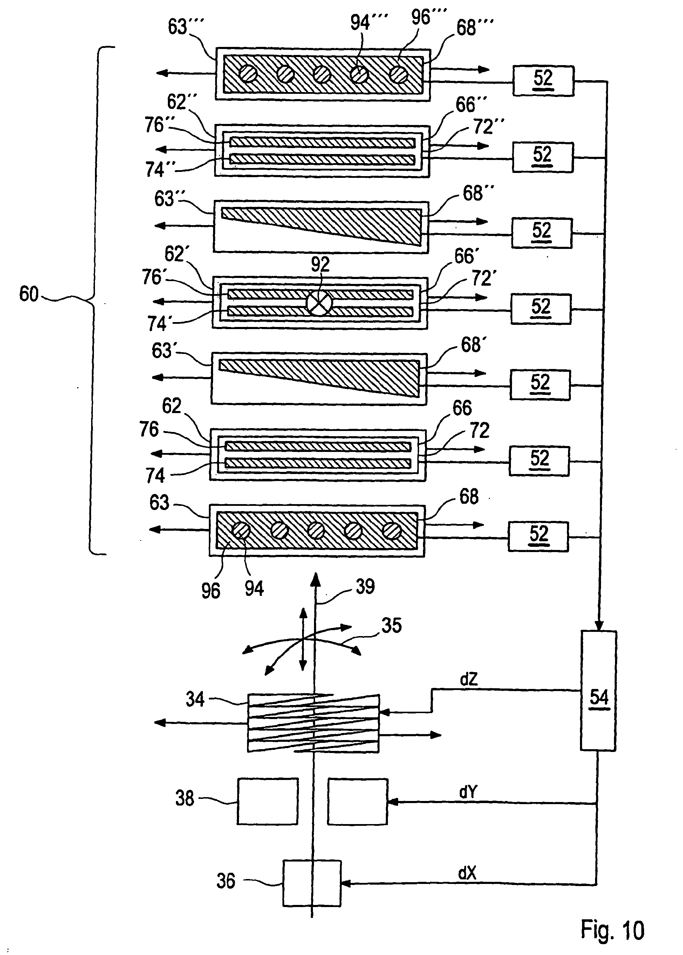

Zur Erfassung der Bewegung können verschiedene Sensoren 52 verwendet werden (siehe insbesondere Fig. 6 und Fig. 10), z.B. das Real-Time-Positioning-Management (RPM) von Varian Medical Systems Inc., Palo Alto, USA, der sogenannte Anzai-Gürtel, z.B. Typ:

AZ-733V von Anzai Medical, Tokio, Japan, ein Stereokamera-basiertes System von Vision RT Ltd, London, England, unter dem Namen Gate RT, implantierte Transponder, z.B. von Calypso Medical Technologies Inc., Seattle, USA, deren Position in drei Dimensionen über elektromagnetische Wechselwirkung bestimmt wird, oder wie bei der Bestrahlungsanlage "Cyberknife", von Accuray Inc., Sunnyvale, USA, bei welchem eine externe Bewegungsmessung durch einen Infrarotmarker mit Fluoroskopie-Daten korrigiert wird. Wenn externe Bewegungssensoren verwendet werden, die nicht unmittelbar der Phantom-Vorrichtung angehören, wird ein Adapter an der Phantom-Vorrichtung angebracht, welcher eine Ankopplung der externen Bewegungssensoren ermöglicht. Zum Beispiel wird an demjenigen Detektor der als Tumor definiert wird, z.B. der zweite Detektor 66', eine Vorrichtung angebracht, welche die RPM Box oder z.B. eine Feder für den Anzai-Sensor aufnehmen kann. Dies hat den Vorteil, dass die zur Bestrahlungseinrichtung gehörende Bewegungserfassung mit validiert werden kann und die Phantom-Vorrichtung eine eigenständige und unabhängige Vorrichtung ist. Für das von der Anmelderin vorgeschlagene Bewegungsphantom eignen sich unter anderem auch industrielle Abstandssensoren auf Basis von Triangulation (z.B. Sick Vertriebs GmbH, Düsseldorf, OD100-35P840).

Für die Bestrahlung mit Bewegungskompensation ist bezugnehmend auf Fig. 5 schematisch dargestellt, wie die mit dem Sensor zur Positionsmessung 52 gewonnenen zeitaufgelösten Positionsdaten in das Therapiekontrollsystem 54 eingespeist werden. Das Therapiekontrollsystem 54 führt mittels der eingespeisten zeitaufgelösten Positionsdaten fortlaufend eine Bewegungskompensation durch Ansteuerung der Scanner-Magnetpaare 36, 38 und des Energiemodulationskeils 34 aus, um die Bewegung des Zielvolumens 48 aktiv zu kompensieren. Für die unterbrochene Bestrahlung wird die Positionsmessung 52 verwendet, um den Strahl gemäß eines durch den Arzt vorgegebenen Bestrahlungsfensterns an- und auszuschalten. Bei Rescanning ist in der einfachsten Ausführungsform keine Positionsmessung notwendig.

Diese Verfahren sind zwar grundsätzlich erprobt, jedoch ist es das Ziel der vorliegenden Erfindung, die Berechnung der RBW-gewichteten Dosis vor allem unter Bewegungseinfluss des Zielvolumens 48 nun experimentell zu validieren.

Dies geschieht z.B. mit der in Fig. 6 dargestellten Phantom-Vorrichtung 60. Die Phantom-Vorrichtung 60 umfasst eine Bewegungseinrichtung in Form eines Bewegungstisches 62 und ein Phantom 64. Das Phantom 64 umfasst einen ersten biologischen Detektor 66 und einen transversal inhomogenen, in diesem Beispiel keilförmigen ersten Absorber 68 strahlaufwärts des ersten biologischen Detektors 66. Der transversal inhomogene erste Absorber 68 kann z.B. statt als Absorberkeil, als Absorbertreppe oder Patientenfeld-spezifischer Absorber ausgebildet sein, welcher vergleichbar zu Kompensatoren für die Ionentherapie mit aufgestreuten Feldern individuell gefräst werden kann. Der erste biologische Detektor 66 umfasst in der in Fig. 6 dargestellten Ausführungsform einen mit Nährmedium gefüllten Behälter 72, in welchen zwei Mikrotiterplatten 74, 76 hintereinander parallel eingesetzt sind. Die Mikrotiterplatten 74, 76 sind durch die dunklen runden Punkte 75 symbolisiert. Der Detektor 66 kann aber ebenfalls eine oder mehrere Gewebeproben enthalten, die einen matrixartigen Aufbau aufweisen, so dass Messpunkte entsprechend den Punkten 75 aufgenommen und analysiert werden können.

Der Bewegungstisch 62 versetzt den Absorber 68 und den ersten biologischen Detektor 66 in eine transversal periodische Bewegung, z.B. eine Schwingung, die in etwa die Frequenz der Atembewegung eines Menschen entspricht. Der Ionenstrahl 39 wird mittels des Therapiekontrollsystems 54 über das Zielvolumen 48 in Form des ersten biologischen Detektors 66 gescannt und der Lasersensor 52 erfasst zeitaufgelöst die Bewegung dV des ersten Detektors 66, um eine in diesem Beispiel nur transversale Bewegungskompensation durchzuführen. In anderen Ausführungen kann der Bewegungstisch 62 durch einen Roboterarm ersetzt werden.

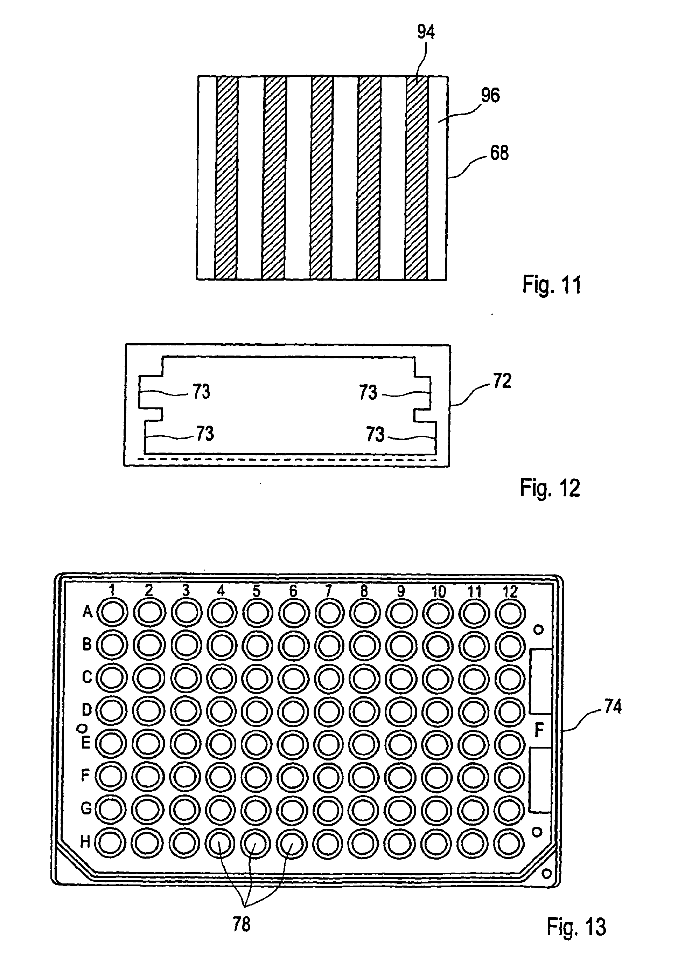

Der Behälter 72 und die Mikrotiterplatte 74 bzw. 76 sind in Fig. 12 bzw. Fig. 13 genauer dargestellt. Die Fig. 13 zeigt die beispielhaft verwendete Mikrotiterplatte NUNC F 96 Mikrowell von der Nunc GmbH & Co. KG. Sie umfasst 96 regelmäßig in 12 Spalten und 8 Zeilen angeordnete Vertiefungen oder Näpfchen 78. Der Abstand des Mittelpunktes eines Näpfchens 78 zum Mittelpunkt des Nachbarnäpfchens 78 beträgt jeweils 9 mm in vertikaler und horizontaler Richtung. Jedes Näpfchen 78 ist konisch geformt und hat einen Öffnungsdurchmesser von 7 mm und am Boden einen Durchmesser von 6,2 mm. Der Boden ist eben und mit einer Wachstumsschicht versehen, um ein Anwachsen der Zellen, z.B. CHO-Zellen zu ermöglichen. Jedes Näpfchen 78 enthält daher eine unabhängige Zellprobe, die einem experimentellen Messpunkt entspricht und daher als "biologischer Detektorpixel" bezeichnet werden kann.

Zur Vorbereitung der Bestrahlung werden ausreichend Zellen in die Näpfchen 78 eingesetzt und entsprechend angezüchtet. Typischerweise enthält ein Näpfchen 78 für die Bestrahlung mehrere Zehntausend Zellen. Nach der Bestrahlung wird die Überlebensrate der Zellen für jedes einzelne Näpfchen 78 separat bestimmt, so dass eine ortsaufgelöste Bestimmung der Überlebensrate der Zellen ermöglicht ist.

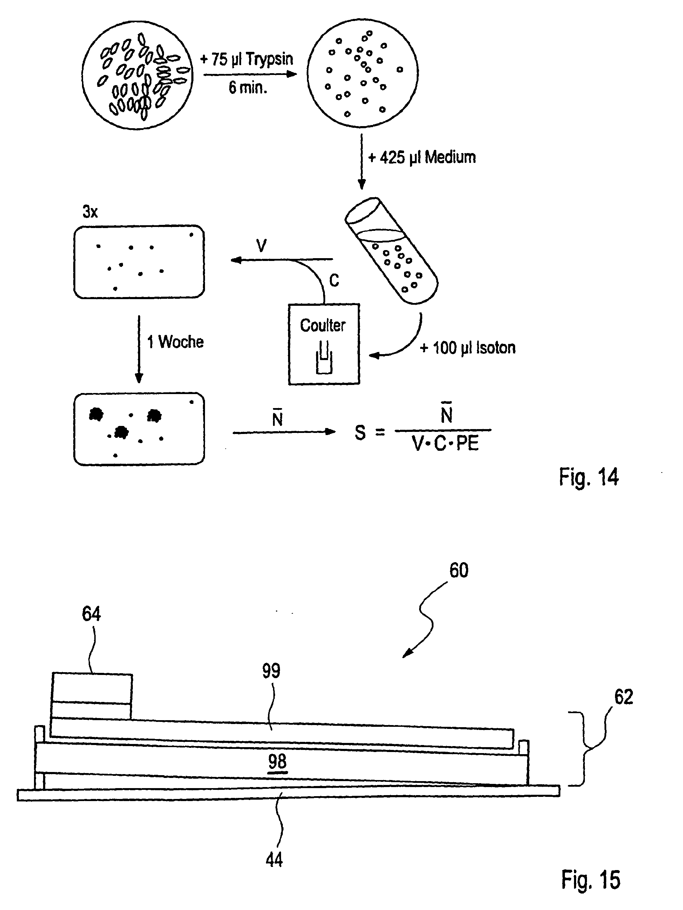

Fig. 14 zeigt ein beispielhaftes Ablaufschema zur Bestimmung des Zellüberlebens. Zunächst werden die Zellen eines Näpfchens zum Ablösen mit Trypsin behandelt. Das Enzym Trypsin löst Eiweißverbindungen auf und führt bei zu langer Behandlung zu Schädigung der Zellen. Die abgelösten und vereinzelten CHO-Zellen werden mit dem Trypsin in einem Medium pippetiert, um die Wirkung des Trypsins abzustoppen. Ein Teil der Zellsuspension wird zur Bestimmung der Zellkonzentration C mit Hilfe eines Coulter-Counters verwendet. Abhängig von der Zellkonzentration und dem zu erwartenden Zellüberleben wird dann jeweils das Volumen V der Zellsuspension in drei T75-Kulturflaschen mit 5 ml Medium gegeben. Danach werden die Zellen für eine Woche in einem Brutschrank gelagert, während die teilungsfähigen Zellen Kolonien bilden. Diese werden nachfolgend gezählt und in einen Überlebenswert S umgerechnet.

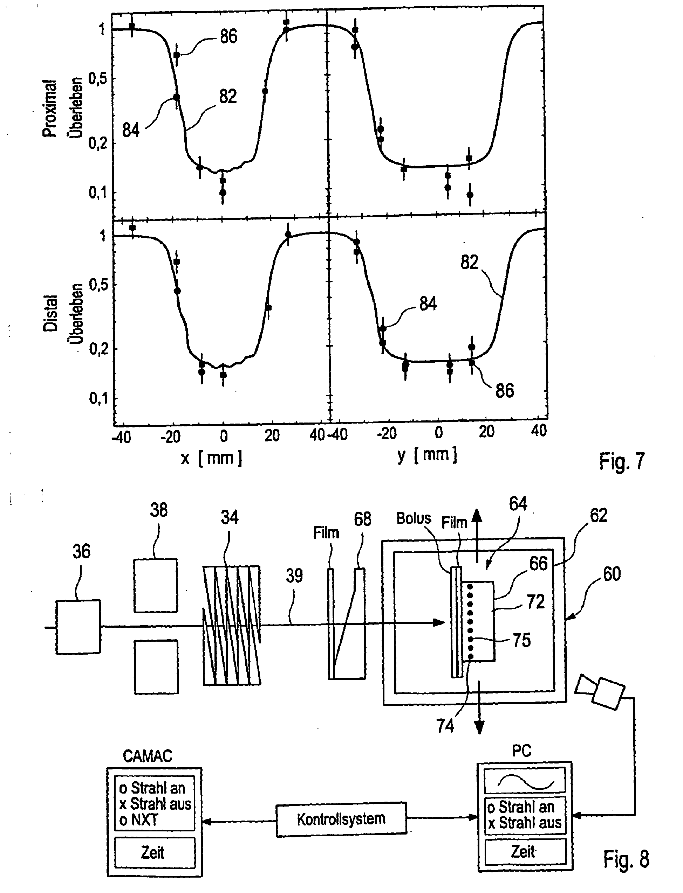

Bezugnehmend auf Fig. 7 ist ein Vergleich der berechneten Überlebensrate mit den experimentell ermittelten Überlebensraten zur erfindungsgemäßen Validierung dargestellt. Die beiden oberen Graphen zeigen die Ergebnisse für die vordere oder proximale Mikrotiterplatte 74 und die beiden unteren Graphen für die hintere oder distale Mikrotiterplatte 76. Die linke Spalte zeigt die Ortsauflösung in X-Richtung und die rechte Spalte in Y-Richtung. Die durchgezogene Linie zeigt die berechnete Funktion 82 für das Zellüberleben, welche erfindungsgemäß validiert werden soll. Die kreisförmigen Messpunkte 84 zeigen nun die stationären, experimentell gemessenen Werte für das Zellüberleben und die quadratischen Messpunkte 86 die experimentell gemessenen Werte für das Zellüberleben bei Bewegung des Phantoms 64 mit Bewegungskompensation. Die Ergebnisse zeigen sehr gute Übereinstimmung der experimentell ermittelten Werte für das Zellüberleben mit der theoretischen Berechnung und das Funktionieren der Bewegungskompensation. Somit ist diese experimentelle Validierung der berechneten Werte höchst vorteilhaft für die Qualität der Bestrahlungsplanung.

Bezugnehmend auf Fig. 8 ist die Bestrahlung des Phantoms 64 dargestellt, wobei der erste Detektor 66 auf dem Bewegungstisch 62 bewegt wird, nicht jedoch der erste Absorber 68, welcher in diesem Beispiel ortsfest ist. In dem in Fig. 8 dargestellten Beispiel wird die Validierungsmessung zwar unter Bewegung des ersten Detektors 66, welcher in diesem Beispiel nur eine Mikrotiterplatte 74 enthält, aber ohne Bewegungskompensation durchgeführt, um im Vergleich zu Fig. 6 und 7 die Vorzüge der Bewegungskompensation nochmals zu zeigen und auch zu zeigen, dass sich die experimentelle Validierung auch ohne Bewegungskompensation anwenden lässt.

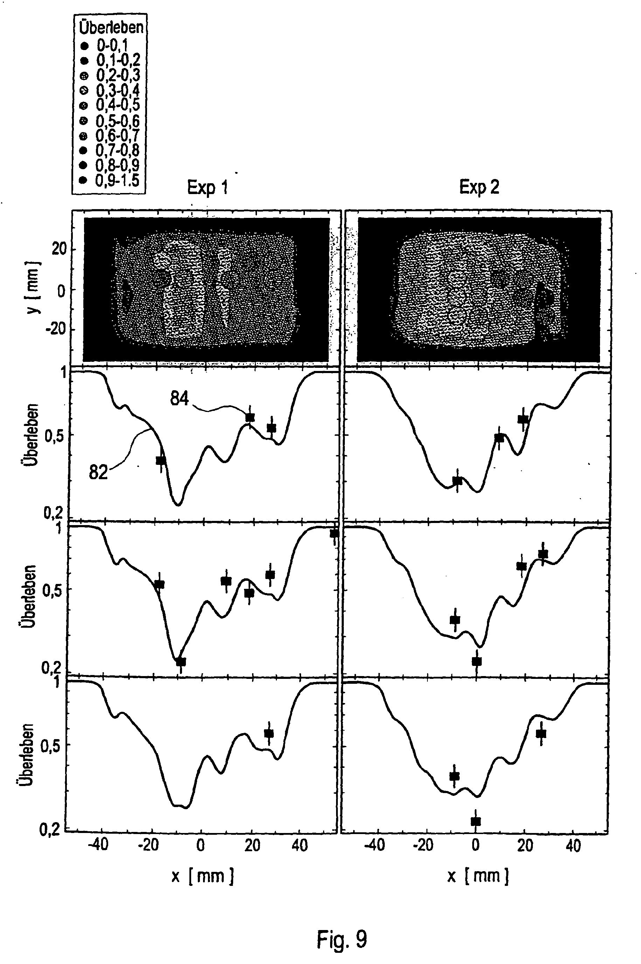

Fig. 9 zeigt wiederum die berechneten Kurven 82 für das Zellüberleben sowie die quadratischen experimentellen Messwerte 88, welche abermals eine gute Übereinstimmung mit den berechneten Kurven zeigen. Die obere Figurenzeile zeigt eine zweidimensionale Darstellung des Zellüberlebens auf dem zweidimensionalen ortsauflösenden ersten biologischen Detektor 66, wobei die Kreise jeweils die experimentell ausgewerteten Messpunkte darstellen. Die drei darunter liegenden Figurenzeilen repräsentieren die drei Zeilen an Messpunkten in den zweidimensionalen Darstellungen der oberen Figurenzeile.

Zusammenfassend kann anhand der bereits durchgeführten Experimente gemäß Fig. 7 und Fig. 9 festgehalten werden, dass sich mit dem erfindungsgemäßen Bewegungs-Phantom 64 unter unterschiedlichen Bestrahlungsbedingungen die verwendeten Algorithmen zur Berechnung der RBW-gewichteten Dosis gut validieren lassen und dass das Phantom 64 grundsätzlich geeignet ist, um eine biologische Dosimetrie für bewegte Zielvolumina 48 vorzunehmen.

Bezugnehmend auf Fig. 10 ist eine besondere Ausführungsform des erfindungsgemäßen Phantoms 64 mit mehreren ortsauflösenden biologischen Detektoren 66, 66', 66" und mehreren Absorbern 68, 68', 68", 68"' dargestellt. Das Phantom 64 umfasst den ersten ortsauflösenden biologischen Detektor 66 mit dem ersten Behälter 72 und mit den biologischen Proben, die als Mikrotiterplatten 74 und 76 ausgebildet sind. In Bezug auf den Ionenstrahl 39 ist strahlaufwärts des ersten Detektors 66 ein erster Absorber 68 angeordnet, welcher jedoch anders ausgestaltet ist als in Fig. 6 und 8. Das Phantom 64 umfasst ferner noch einen zweiten und dritten transversal inhomogenen Absorber 68', 68", in diesem Beispiel jeweils keilförmig und einen weiteren rückwärtigen strukturierten Absorber 68"' sowie einen zweiten und dritten ortsauflösenden biologischen Detektor 66', 66". Die biologischen Detektoren 66, 66', 66" weisen jeweils getrennte Behälter 72, 72', 72" zum Einsetzen der jeweiligen Mikrotiterplatten 74, 76; 74', 76'; 74", 76" auf. Hierdurch können in den unterschiedlichen biologischen Detektoren unterschiedliche Nährlösungen eingefüllt werden und unterschiedliche Zelltypen und/oder Gewebetypen gezüchtet werden.

Die Absorber 68, 68', 68", 68"' sowie die Detektoren 66, 66', 66" sind in diesem Beispiel mittels unabhängiger Bewegungseinrichtungen 62, 62', 62"; 63, 63', 63", 63"' alle unabhängig voneinander bewegbar, um eine hochkomplexe realistische Bewegungssimulation des zu bestrahlenden Körpers erreichen zu können. Jedem Absorber und Detektor ist jeweils ein Bewegungssensor 52 zugeordnet, welcher die Bewegungsdaten jedes einzelnen Absorbers und Detektors zeitaufgelöst und zeitlich korreliert zum Beschleunigungsvorgang mit einem Datenerfassungssystem 99 protokolliert. Diese Daten fließen in die Dosisberechnung ein, anhand derer das zu erwartende Überlebensniveau S pro Detektorelement bestimmt wird. Parallel können die Daten an das Therapiekontrollsystem 54 übermittelt werden, um falls gewünscht, eine bewegungskompensierte Bestrahlung oder unterbrochene Bestrahlung und Validierung durchführen zu können. Das Therapiekontrollsystem steuert das dreidimensionale Scannen (dX, dY, dZ) mittels Ansteuerung der Magnetpaare 36 und 38 sowie der aktiven Energievariation durch das Synchrotron. Das dreidimensionale Scannen ist mit den Pfeilen 35 symbolisiert. Mit der Mehrzahl an unabhängig voneinander bewegbaren Absorbern 68, 68', 68", 68"' und/oder ortsauflösenden biologischen Detektoren 66, 66', 66" kann eine differenzielle Bewegung unterschiedlicher Gewebeschichten im Körper des Patienten, z.B. eine komplexe Bewegung des Gewebes beim Atmen simuliert werden.

Zusätzlich weist das Phantom 64 noch eine Dreheinrichtung mit Drehpunkt 92 auf, um den das Phantom 64 drehbar gelagert ist. Hiermit können z.B. zwei oder mehr Felder aus unterschiedlichen Winkeln eingestrahlt werden, ohne eine Veränderung des Aufbaus des Phantoms 64 vornehmen zu müssen. Hierzu ist der weitere Absorber 68"' auf der dem ersten Absorber 68 gegenüberliegenden Seite des ersten biologischen Detektors 66 vorgesehen. Somit ist der erste biologische Detektor 66, welcher den zu bestrahlenden Tumor repräsentiert, zwischen dem ersten Absorber 68 und dem weiteren Absorber 68"' angeordnet, so dass der erste Absorber 68 die Rippen auf der Vorderseite des Tumors und der weitere Absorber 68"' die Rippen auf der Rückseite des Tumors simulieren kann, oder vice versa.

Bezug nehmend auf die Fig. 10 und 11 ist der erste Absorber 68 intern strukturiert. Der erste Absorber 68 weist in diesem Beispiel Stäbe 94 mit höherem Absorptionsvermögen auf, welche periodisch nebeneinander angeordnet sind. Die Stäbe (hier aus Teflon gefertigt) 94 sind in einem Block aus einem Material 96 mit niedrigerem Absorptionsvermögen, hier PMMA eingebettet. Die Stäbe 94 simulieren hierbei menschliche Rippen und das Material 96 zwischen den Stäben menschliches Gewebe zwischen den Rippen. Der weitere rückwärtige Absorber 68"' ist ähnlich wie der erste Absorber 68 strukturiert und simuliert die hinteren Rippen. Der hintere Absorber 68"' kann ein anderes Verhältnis der Dichten zwischen den Stäben 94"' und dem Zwischenmaterial 96"' als der vordere Absorber 68 aufweisen. Somit kann mit dem Absorber 68 eine relativ realistische Simulation des menschlichen Brustkorbs im Bereich der Rippen erzielt werden.

Bezug nehmend auf Fig. 12 weist der Behälter 72 Führungsnuten 73 auf, in welche die Mikrotiterplatten 74, 76 hintereinander stehend einschiebbar sind. Beim Einschieben der Mikrotiterplatten 74, 76 sollte darauf geachtet werden, dass keine Luftblasen in den Näpfchen 78 entstehen. Nach dem Einschieben der Mikrotiterplatten wird der Behälter 72 dann soweit mit Nährmedium gefüllt, dass alle zu bestrahlenden Näpfchen 78 der Mikrotiterplatten 74, 76 mit Nährmedium gefüllt sind. Die Mikrotiterplatten 74, 76 werden dabei unverschlossen in den Behälter 72 eingesetzt, um die Entstehung von Luftblasen in den Näpfchen zu verhindern. Dadurch wird ein negativer und undefinierbarer Einfluss von Luftblasen auf die Strahlreichweite vermieden. Ferner kann eine Unterbrechung der Fehlversorgung mit Nährmedium vermieden werden, was ebenfalls das Versuchsergebnis verfälschen könnte. Der Behälter 72 gestattet das Einfügen zweier Mikrotiterplatten hintereinander, so dass nicht nur ein zweidimensional lateral ortsauflösender biologischer Detektor bereitgestellt wird, sondern sogar jeweils zwei Messpunkte in longitudinaler Richtung hintereinander bereitgestellt sind. Die beiden Mikrotiterplatten können in einer Flucht hintereinander oder beispielsweise mit Versatz von je einem halben Näpfchen in den Behälter 72 hineingestellt werden. Der Behälter 72 besteht vorzugsweise aus PMMA, welches sich gut desinfizieren lässt und wenig schwere Elemente enthält, die den lonenstrahl zusätzlich aufstreuen könnten.

Fig. 13 zeigt ein Beispiel einer Mikrotiterplatte 74 mit 96 matrixartig angeordneten Näpfchen 78. Die Verwendung von Mikrotiterplatten 74, 76 mit Näpfchen 78 ist insbesondere für ein bewegtes Phantom 64 vorteilhaft, da die Zellen am Boden der Näpfchen 78 adhärent anwachsen und damit auch bei Bewegung des biologischen Detektors 66 die biologischen Zellen vor allzu starker Umspülung oder Strömung geschützt sind.

Fig. 15 zeigt einen beispielhaften Aufbau eines Bewegungstisches 62 mit einem stationären Plattform 98 und einer darauf beweglich angeordneten Plattform 99, welche zusammen auf der Patientenliege 44 aufgestellt sind. Auf der Bewegungsplattform 99 ist das Phantom 64 aufgestellt, welches mit dem Bewegungstisch 62 bewegt wird. Der Aufbau in Fig. 15 entspricht hierbei dem einfachen Aufbau in Fig. 8.

Die stationäre Plattform 98 des Bewegungstisches 62 ist aus einer Trovidur-Platte gefertigt und ist leicht geneigt, um die Neigung des Strahls am Therapieplatz von in diesem Beispiel 2,2° auszugleichen. Die bewegliche Plattform 99 aus PMMA kann in diesem Beispiel eine eindimensionale sinusförmige Bewegung durchführen, welche durch einen Motor erzeugt wird und über ein verstellbares Exzenterrad auf die Plattform 99 übertragen wird. Die Amplitude der Bewegung ist einstellbar, in diesem Beispiel zwischen 1 mm und 25 mm. Ferner ist die Periode der Bewegung einstellbar, in diesem Beispiel zwischen 2,5 s und 6,5 s. Die Bewegungseinrichtung 62 ist durch ein externes Signal steuerbar, so dass zum Beispiel die Bewegung synchron zum Strahlungsbeginn gestartet werden kann, um z.B. eine bestimmte Anfangsphase des Tisches einzustellen.

In diesem Beispiel erfolgt die Messung der Bewegung mit einem Lasersensor 52 OD 100-35P840, der SICK Vertriebs-GmbH, Deutschland. Der Lasersensor 52 ist an der stationären Platte 98 befestigt und misst die Bewegung der beweglichen Plattform 99.

Zusammenfassend gestattet die Erfindung eine biologische Dosimetrie unter Bewegungseinfluss, ggfs. mit differenzieller Bewegung zwischen einem oder mehreren biologischen Detektoren 66, 66', 66" und einem oder mehreren Absorbern 68, 68', 68", 68"'.

In der Partikeltherapie muss insbesondere bei Ionen, die schwerer sind als Protonen, von einer erhöhten biologischen Wirksamkeit ausgegangen werden, d.h. die absorbierte Dosis wird in Abhängigkeit von dem Dosislevel, der Energie und spektralen Zusammensetzung des Teilchenfeldes und dem biologischen System mit einem Faktor gewichtet, um die biologisch effektive Dosis zu beschreiben, die in neuerer Terminologie als RBW-gewichtete Dosis bezeichnet wird. Die erhöhte biologische Wirkung muss in der Bestrahlungsplanung berücksichtigt werden, was durch komplexe Modellierung erfolgt. Diese komplexe biologische Modellierung kann mit dem erfindungsgemäßen Phantom experimentell in vitro validiert werden.

Mit der vorliegenden Erfindung kann also ein Verfahren zur Validierung der Therapieplanung für gescannte Teilchenstrahlen und ein Bewegungsphantom bereitgestellt werden, das die Messung der biologischen Wirkung der Strahlung an Zellkulturen in vitro erlaubt. Insbesondere wurde ein Verfahren und ein Phantom entwickelt, welches eine Anbindung an das Therapiekontrollsystem 54 einer Partikeltherapie-Beschleunigeranlage 30 und eine routinemäßige und verlässliche Durchführung von Experimenten zum Zellüberleben auch bei Bestrahlung mit Bewegungskompensation erlaubt. Mit dem erfindungsgemäßen Bewegungsphantom kann das Verfahren zur Bestrahlung von intra-fraktionär bewegten Tumoren einer Partikeltherapie-Beschleunigeranlage getestet und der Algorithmus zur Berechnung der RBW-gewichteten Dosis validiert werden.

Es ist dem Fachmann ersichtlich, dass die vorstehend beschriebenen Ausführungsformen beispielhaft zu verstehen sind, und die Erfindung nicht auf diese beschränkt ist, sondern in vielfältiger Weise variiert werden kann, ohne die Erfindung zu verlassen. Ferner ist ersichtlich, dass die Merkmale unabhängig davon, ob sie in der Beschreibung, den Ansprüchen, den Figuren oder anderweitig offenbart sind auch einzeln wesentliche Bestandteile der Erfindung definieren, selbst wenn sie zusammen mit anderen Merkmalen gemeinsam beschrieben sind.

**Patentansprüche**

1.  Phantom-Vorrichtung (60) zur experimentellen in-vitro-Validierung von Bestrahlungen unter Bewegungseinfluss unter Berücksichtigung der biologisch effektiven Dosis, insbesondere für therapeutische Bestrahlungen an einer Partikeltherapie-Beschleunigeranlage (30), umfassend
    ein Phantom (64), welches einen ersten biologischen Detektor (66) mit einer ersten biologischen Probe (74) umfasst, wobei die erste biologischen Probe eine Mehrzahl von Zucht- und Bestrahlungselementen (78) mit jeweils separaten biologischen Proben aufweist, so dass der erste biologische Detektor (66) als ortsauflösender biologischer Detektor ausgebildet ist,
    eine erste Bewegungseinrichtung (62) für den ersten biologischen Detektor mittels welcher der erste biologische Detektor (66) bewegbar ist, um die Bewegung des Zielvolumens (48) mit dem Phantom (64) zu simulieren, **gekennzeichnet durch**
    einen transversal inhomogenen ersten Absorber (68) strahlaufwärts des ersten biologischen Detektors (66) und eine zweite Bewegungseinrichtung (63) für den ersten Absorber mittels welcher der erste Absorber (68) unabhängig von dem ersten biologischen Detektor (66) bewegbar ist, um die Bewegung des Gewebes strahlaufwärts des zielvolumens (48) mit dem Phantom zu simulieren.

**2.** Phantom-Vorrichtung (60) nach einem der vorstehenden Ansprüche, wobei der erste Absorber (68) mehrere Bereiche (94) mit höherem Strahlabsorptionsvermögen umfasst, zwischen welchen Material (96) mit geringerem Strahlabsorptionsvermögen angeordnet ist.

**3.** Phantom-Vorrichtung (60) nach einem der vorstehenden Ansprüche, ferner umfassend einen zweiten biologischen Detektor (66') und eine dritte Bewegungseinrichtung (62'), wobei der zweite biologische Detektor (66') mittels der dritten Bewegungseinrichtung bewegbar ist.

**4.** Phantom-Vorrichtung (60) nach einem der vorstehenden Ansprüche, ferner umfassend eine Dreheinrichtung (92), mittels welcher das Phantom um eine Achse quer zur Richtung des Strahls aus der Partikeltherapie-Beschleunigeranlage (30) drehbar ist.

**5.** Phantom-Vorrichtung (60) nach einem der vorstehenden Ansprüche, ferner umfassend einen weiteren Absorber (68''') und eine weitere Bewegungseinrichtung (63''') für den weiteren Absorber (68'''), wobei der weitere Absorber (68''') auf der gegenüberliegenden Seite des ersten biologischen Detektors (66) angeordnet ist, als der erste Absorber (68), so dass der erste biologische Detektor bezogen auf den Strahl aus der Partikeltherapie-Beschleunigeranlage (30) zwischen dem ersten Absorber (68) und dem weiteren Absorber (68''') angeordnet ist.

**6.** Phantom-Vorrichtung (60) nach einem der vorstehenden Ansprüche, ferner umfassend einen Bewegungssensor (52), welcher die Bewegung des Phantoms (64) erfasst und eine Steuereinrichtung (54) für die Partikeltherapie-Beschleunigeranlage (30) zur bewegungskompensierten Bestrahlung des Phantoms (64).

**7.** Phantom-Vorrichtung (60) nach einem der vorstehenden Ansprüche, wobei das Phantom (64) einen ersten biologischen Detektor (66) mit einer ersten biologischen Probe (74) umfasst, welche eine Mehrzahl von Zucht- und Bestrahlungselementen mit jeweils separaten biologischen Proben, insbesondere biologische Zellkulturen aufweist, wobei die erste biologische Probe (74) als eine Platte mit einer Mehrzahl von näpfchenförmigen Vertiefungen (78) ausgebildet ist, wobei die Vertiefungen (78) eine Oberfläche aufweisen, welche zum Anwachsen der biologischen Proben, insbesondere der biologischen Zellkulturen hergerichtet ist, so dass die biologischen Proben, insbesondere biologische Zellkulturen adhärent innerhalb der Vertiefungen (78) anwachsen können.

**8.** Phantom-Vorrichtung (60) nach Anspruch 7, wobei bei dem Phantom (64) die erste biologische Probe (74) als eine erste Mikrotiterplatte (74) ausgebildet ist, wobei der erste biologische Detektor (66) einen ersten Behälter (72) für Flüssigkeit aufweist, in welchen die erste Mikrotiterplatte (74) einsetzbar und wieder entnehmbar ist, derart dass die erste Mikrotiterplatte (74) beidseits von der Flüssigkeit umgeben ist, wenn die erste Mikrotiterplatte (74) in dem Behälter (72) eingesetzt und der Behälter (72) mit der Flüssigkeit gefüllt ist.

**9.** Phantom-Vorrichtung (60) nach Anspruch 7 oder 8, wobei das Phantom (64) ferner einen zweiten biologischen Detektor (66') mit einem zweiten Behälter (72') für Flüssigkeit umfasst, in welchen eine weitere Mikrotiterplatte (74') einsetzbar und wieder entnehmbar ist, wobei der erste und zweite Behälter (72, 72') hintereinander angeordnet sind.

**Claims**

**1.** Phantom device (60) for the experimental in-vitro validation of irradiations under the influence of movement, taking into account the biologically effective dose, in particular for therapeutic irradiations at a particle therapy acceleration unit (30), comprising a phantom (64) which comprises a first biological detector (66) with a first biological sample (74), wherein the first biological sample has a plurality of culture and irradiation elements (78) with separate biological samples in each case, so that the first biological detector (66) is formed as a spatial, biological detector, a first movement device (62) for the first biological detector, by means of which the first biological detector (66) is movable, to simulate with the phantom (64) the movement of the target volume (48), **characterized by** a transversally inhomogeneous first absorber (68) upstream of the first biological detector (66) and a second movement device (63) for the first absorber through which the first absorber (68) can be moved independently of the first biological detector (66), to simulate with the phantom (64) the movement of the tissue upstream of the target volume (48).

**2.** Phantom device (60) according to claim 1, wherein the first absorber (68) comprises a plurality of regions (94) with a higher radiation absorption capacity, between which material (96) with a lower radiation absorption capacity-is disposed.

**3.** Phantom device (60) according to one of the above claims, further comprising a second biological detector (66') and a third movement device (62'), wherein the second biological detector (66') can be moved by means of the third movement device.

**4.** Phantom device (60) according to one of the above claims, further comprising a rotation device (92) through which the phantom can be rotated about an axis transverse to the direction of the beam from the particle therapy acceleration unit (30).

**5.** Phantom device (60) according to one of the above claims, further comprising a further absorber (68''') and a further movement device (63''') for the further absorber (68'''), wherein the further absorber (68''') is disposed on the opposite side of the first biological detector (66) to the first absorber (68) so that the first biological detector.relative to the beam from the particle therapy acceleration unit (30) is disposed between the first absorber (68) and the further absorber (68''').

**6.** Phantom device (60) according to one of the above claims, further comprising a movement sensor (52), which detects the movement of the phantom (64) and a control device (54) for the particle therapy acceleration unit (30) for a movement-compensated irradiation of the phantom (64).

**7.** Phantom device (60) according to one of the above claims, wherein the phantom (64) comprises a first biological detector (66) with a first biological sample (74), which has a plurality of culture and irradiation elements with separate biological samples in each case, in particular biological cell cultures, wherein the first biological sample (74) is formed as a plate with a plurality of saucer cup-shaped wells (78), wherein the wells (78) have a surface that is designed for the growth of biological samples, in particular the biological cell cultures, so that the biological samples, in particular biological cell cultures, can grow adhering within the wells (78).

**8.** Phantom device (60) according to claim 7, wherein for the phantom (64) the first biological sample (74) is formed as a first microtitre plate (74), wherein the first biological detector (66) has a first container (72) for a liquid, in which the first microtitre plate (74) can be inserted and removed such that the first microtitre plate (74) is surrounded on both sides by the liquid when the first microtitre plate (74) is inserted in the container (72) and the container (72) is filled with the liquid.

**9.** Phantom device (60) according to claim 7 or 8, wherein the phantom (64) further comprises a second biological detector (66') with a second container (72') for a liquid, in which a further microtitre plate (74') can be inserted and removed again, wherein the first and second containers (72, 72') are arranged one behind the other.

**Revendications**

**1.** Dispositif fantôme (60) destiné à la validation expérimentale in vitro d'irradiations sous l'effet de mouvements en tenant compte de la dose biologiquement effective, en particulier pour des irradiations thérapeutiques sur un accélérateur de particules de radiothérapie (30), comprenant
un fantôme (64) qui comprend un premier détecteur biologique (66) avec un premier échantillon biologique (74), le premier échantillon biologique présentant une pluralité d'éléments de culture et d'irradiation (78) avec des échantillons biologiques respectivement distincts, de sorte que le premier détecteur biologique (66) est conçu en tant que détecteur biologique à résolution locale,
un premier dispositif de mouvement (62) pour le premier détecteur biologique, au moyen duquel le premier détecteur biologique (66) est déplaçable afin de simuler avec le fantôme (64) le mouvement du volume cible (48), **caractérisé par**
un premier absorbeur (68) transversalement inhomogène en amont du premier détecteur biologique (66) et un deuxième dispositif de mouvement (63) pour le premier absorbeur, au moyen duquel le premier absorbeur (68) est déplaçable indépendamment du premier détecteur biologique (66) afin de simuler avec le fantôme le mouvement du tissu en amont du volume cible (48).

**2.** Dispositif fantôme (60) selon la revendication 1, dans lequel le premier absorbeur (68) comprend plusieurs zones (94) avec fort pouvoir absorbant de rayonnement, entre lesquelles une matière (96) est placée avec plus faible pouvoir absorbant de rayonnement.

**3.** Dispositif fantôme (60) selon l'une des revendications précédentes, comprenant en outre un deuxième détecteur biologique (66') et un troisième dispositif de mouvement (62'), le deuxième détecteur biologique (66') étant déplaçable au moyen du troisième dispositif de mouvement.

**4.** Dispositif fantôme (60) selon l'une des revendications précédentes, comprenant en outre un dispositif de rotation (92) au moyen duquel le fantôme peut pivoter autour d'un axe transversal par rapport à la direction du rayonnement issu de l'accélérateur de particules de radiothérapie (30).

**5.** Dispositif fantôme (60) selon l'une des revendications précédentes, comprenant en outre un absorbeur supplémentaire (68''') et un dispositif de mouvement supplémentaire (63''') pour l'absorbeur supplémentaire (68'''), l'absorbeur supplémentaire (68''') étant placé du côté opposé du premier détecteur biologique (66) par rapport au premier absorbeur (68), de sorte que relativement au rayonnement issu de l'accélérateur de particules de radiothérapie (30), le premier détecteur biologique est placé entre le premier absorbeur (68) et l'absorbeur supplémentaire (68''').

6. Dispositif fantôme (60) selon l'une des revendications précédentes, comprenant en outre un capteur de mouvement (52) qui capte le mouvement du fantôme (64), et un dispositif de commande (54) pour l'accélérateur de particules de radiothérapie (30) afin d'obtenir une irradiation du fantôme (64) compensée en mouvement.

7. Dispositif fantôme (60) selon l'une des revendications précédentes, dans lequel le fantôme (64) comprend un premier détecteur biologique (66) avec un premier échantillon biologique (74) qui comprend une pluralité d'éléments de culture et d'irradiation avec des échantillons biologiques respectivement distincts, en particulier des cultures cellulaires biologiques, sachant que le premier échantillon biologique (74) est conçu en tant que plaque avec une pluralité d'évidements (78) en forme de godet, et que les évidements (78) présentent une surface préparée pour la croissance des échantillons biologiques, en particulier des cultures cellulaires biologiques, de sorte que les échantillons biologiques, en particulier des cultures cellulaires biologiques, peuvent croître par adhérence à l'intérieur des évidements (78).

8. Dispositif fantôme (60) selon la revendication 7, sachant que dans le fantôme (64), le premier échantillon biologique (74) est conçu en tant que première plaque de microtitration (74), que le premier détecteur biologique (66) présente un premier récipient (72) pour liquides, dans lequel la première plaque de microtitration (74) peut être placée puis retirée, de manière telle que la première plaque de microtitration (74) est entourée des deux côtés par le liquide lorsque la première plaque de microtitration (74) est placée dans le récipient (72) et le récipient (72) est rempli de liquide.

9. Dispositif fantôme (60) selon la revendication 7 ou 8, dans lequel le fantôme (64) comprend en outre un deuxième détecteur biologique (66') avec un deuxième récipient (72') pour liquides, dans lequel une autre plaque de microtitration (74') peut être placée puis retirée, le premier et le deuxième récipients (72, 72') étant disposés l'un derrière l'autre.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

$$S = \frac{\bar{N}}{V \cdot C \cdot PE}$$

Fig. 14

Fig. 15

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007045879
- WO 2007104520 A1
- US 20080298540 A1
- DE 102007045879 A1

**In der Beschreibung aufgeführte Nicht-Patentliteratur**

- **S.O. GRÖTZINGER.** Volume Conformal Irradiation of Moving Target Volumes with scanned ionbeams. *Dissertationen,* 2004
- **C. BERT.** Bestrahlungsplan für bewegte Zielvolumina in der Tumortherapie mit gescanntem Kohlenstoffstrahl. *DISSERTATIONEN,* 2006